(19) Europäisches Patentamt

European Patent Office

Office européen des brevets

(11) EP 0 571 920 B1

(12) **EUROPÄISCHE PATENTSCHRIFT**

(45) Veröffentlichungstag und Bekanntmachung des Hinweises auf die Patenterteilung:
**15.01.1997 Patentblatt 1997/03**

(51) Int. Cl.$^6$: **C07C 17/00**, C07C 17/26, C07C 19/08

(21) Anmeldenummer: **93108352.1**

(22) Anmeldetag: **24.05.1993**

(54) **Verfahren zur Herstellung von Fluorkohlenwasserstoffen**

Process for the preparation of fluoro hydro carbons

Procédé pour la préparation d'hydrocarbures fluorés

(84) Benannte Vertragsstaaten:
**BE DE ES FR GB GR IT NL**

(30) Priorität: **26.05.1992 DE 4217398**

(43) Veröffentlichungstag der Anmeldung:
**01.12.1993 Patentblatt 1993/48**

(73) Patentinhaber: **SOLVAY (Société Anonyme)**
**B-1050 Bruxelles (BE)**

(72) Erfinder: **Jansen, Rolf-Michael, Dr.**
**W-6233 Kelkheim (DE)**

(74) Vertreter: **Jacques, Philippe et al**
**Solvay S.A.**
**Département Propriété Industrielle**
**310, rue de Ransbeek**
**1120 Bruxelles (BE)**

(56) Entgegenhaltungen:
**EP-A- 0 442 087          WO-A-90/08748**

- **CHEMISTRY LETTERS. Nr. 10, 1991, TOKYO JP Seiten 1825 - 1826 SATOSHI TOMIOKA ET AL. 'A Novel Hydrodechlorinative Dimerization of Chlorofluorocarbons over Supported Ni Catalysts'**

## Beschreibung

Die vorliegende Erfindung betrifft die Herstellung von Fluorkohlenwasserstoffen.

Fluorkohlenwasserstoffe werden als Ersatzstoffe für die ozonschädigenden vollhalogenierten Fluorchlorkohlenwasserstoffe (FCKW) eingesetzt. So ist beispielsweise 1,1,1,4,4,4-Hexafluorbutan (R 356) ein Ersatzstoff für Trichlorfluormethan (R 11).

Für diese Verbindung sind bereits spezielle Herstellverfahren bekannt. R 356 entsteht z.B. bei der katalytischen Hydrierung von 1,1,1,4,4,4-Hexafluorbuten-2 (R.N. Hazeldine, J.Chem. Soc., 1952, Seite 2504). Dieses Ausgangsmaterial ist jedoch toxisch und nur auf unwirtschaftliche Weise herstellbar.

Nach Tamioka et al., Chemistry Letters, Seite 1825-1826, 1991, Chemical Society of Japan, kann man $F_3C$-$CCl_3$ (R113a) in Gegenwart von $H_2$ an Ni-Katalysatoren zu 1,1,1,4,4,4-Hexafluor-2,3-dichlorbuten-2 (R 1316) umsetzen. Nach den Angaben in dieser Veröffentlichung entsteht dabei aus dem R 113a kein R 356. Aus dem nach diesem Prozeß herstellbaren, sehr giftigen R 1316 kann R 356 laut DE-OS 3 735 467 (entsprechend US-PS 4 902 839 und US-PS 4 954 666) durch katalytische Hydrierung in Gegenwart einer Base hergestellt werden.

WO-90 087 48 offenbart ein Verfahren zur katalytischen Dehydrohalogenierung von Fluorhalogenkohlenstoffen und Fluorhalogenkohlenwasserstoffen, gekennzeichnet durch die Verwendung eines Rhenium enthaltenden Katalysators.

EP-A-0 442 087 offenbart ein Verfahren zur Herstellung gesättigter, fluorhaltiger und chlorfreier Kohlenwasserstoffe, indem man ungesättigte fluor- und chlorhaltige Kohlenwasserstoffe bei über 80 °C in der Gasphase katalytisch hydriert.

Überraschenderweise wurde nun gefunden, daß R 113a durch katalytische Hydrierung direkt in R 356 umgewandelt werden kann und daß ähnliche Ausgangsmaterialien analog "hydrierend dimerisiert" werden.

Gegenstand der Erfindung ist ein Verfahren zur Herstellung von gesättigten Fluorkohlenwasserstoffen, dadurch gekennzeichnet, daß man mindestens einen Fluorkohlenwasserstoff der Formel (I)

$$R_f\text{-}CH_nCl_cBr_dJ_e \qquad (I)$$

worin $R_f = C_aF_{(2a-b)+1}H_b$ mit $a = 1 - 4$, $b = 0 - 4$,
$n = 0 - 2$
$c = 0 - 3$
$d = 0 - 3$
$e = 0 - 3$
$c+d+e = 3-n$ ist,

hydrierend dimerisiert, indem man ihn mit Wasserstoff in Gegenwart von mindestens einem Katalysator, der ein Element der I., II. oder VIII. Nebengruppe enthält, in einem Reaktionsgefäß umsetzt, das eine Zone A mit einer Temperatur von 350 bis 500°C und eine Zone B mit einer Temperatur von 150 bis 300°C enthält, wobei man die Reaktanten zuerst durch Zone A und dann durch Zone B leitet.

Vorzugsweise ist $c = 1 - 3$ und $d = e = 0$. Stets gilt natürlich $2a-b \geq 0$, da es sich bei den Verbindungen (I) um Fluorkohlenwasserstoffe handelt, die daher mindestens ein F-Atom enthalten.

Für das erfindungsgemäße Verfahren als Ausgangsmaterialien geeignete Fluorkohlenwasserstoffe der Formel (I) sind beispielsweise 1-Chlor-2,2,2-trifluorethan (R 133a), 1,1-Dichlor-2,2,2-trifluorethan (R 123), 1,1,1-Trichlor-2,2,2-trifluorethan (R 113a) oder 1,1-Dichlor-2,2,3,3,3-pentafluorpropan. Bevorzugt wird 1,1,1-Trichlor-2,2,2-trifluorethan (R 113a) eingesetzt; in diesem Fall entsteht R 356 in hoher Ausbeute und Selektivität. Man kann aber auch Mischungen aus zwei oder mehr Fluorkohlenwasserstoffen der Formel (I) einsetzen.

Als Katalysatoren kommen für das erfindungsgemäße Verfahren Elemente der I. bis VIII. Nebengruppe des Periodensystems in Frage, vor allem Elemente der I., II. oder VIII. Nebengruppe. Die Elemente können in metallischer Form oder in Form von Verbindungen (z. B. als Oxide oder Hydroxide) vorliegen, und zwar mit oder ohne Trägermaterialien, wie Aktivkohle, $SiO_2$, $Al_2O_3$, MgO, $TiO_2$, $ZrO_2$. Die Katalysatoren werden bevorzugt auf Trägermaterialien eingesetzt. Bevorzugte Trägermaterialien sind Aktivkohle, $SiO_2$ oder $Al_2O_3$.

Die Reaktanten durchlaufen zuerst Zone A und dann Zone B. Innerhalb der Zone A kann die Temperatur konstant sein, sie kann aber auch vom Anfang der Zone zum Ende hin abfallen oder steigen. Dasselbe gilt für die Zone B. Vorzugsweise fällt die Temperatur in beiden Zonen von Anfang zum Ende hin ab, wobei dann am Ende der Zone A eine um mindestens 50°C höhere Temperatur herrscht als am Anfang der anschließend durchlaufenen Zone B.

Die Länge der Zonen A und B beträgt im allgemeinen jeweils 10 % - 50 % der Gesamtlänge des (länglichen) Reaktors. Im Falle eines Rohrreaktors von 2 m Länge hat also z. B. jede der beiden Zonen eine Länge von 0,2 bis 1 m.

Man kann in Zone A und B den gleichen Katalysator verwenden, dann setzt man vorzugsweise Fe, Co, Cu oder Ni ein, insbesondere Ni. Man kann auch, was besonders bevorzugt ist, zwei verschiedene Katalysatoren in den zwei Zonen verwenden; in diesem Fall setzt man in Zone A vorzugsweise Fe, Co, Cu oder Ni ein, insbesondere Ni, und in der Zone B vorzugsweise Ni, Ru, Rh, Pd oder Pt, insbesondere Rh.

Das erfindungsgemäße Verfahren wird im allgemeinen bei Drücken von 1 bis 200 bar, bevorzugt bei 1 bis 25 bar durchgeführt. Das Wasserstoff-Edukt-Verhältnis liegt im allgemeinen bei 1:1 bis 20:1, bevorzugt bei 4:1 bis 10:1.

Die Erfindung soll durch nachfolgende Beispiele erläutert werden.

Beispiel 1

Durch ein Reaktorrohr (L = 2000 mm, $\varnothing$ = 40 mm), das mit 500 g eines Ni/SiO$_2$-Katalysators (Verhältnis Ni/SiO$_2$ = 1:5, Herstellung siehe Ueda et al., Chem. Letters, 1990, Seite 879 - 880) gefüllt war, wurden R 113a (50 ml/h; Dosierung erfolgte flüssig mit Hilfe einer Membranpumpe) und H$_2$ geleitet, wobei der Wasserstoffstrom mit einem thermischen Massendurchflußregler so eingestellt wurde, daß das Molverhältnis H$_2$/R 113a 6:1 betrug. Durch zwei unabhängig gesteuerte Öfen werden in dem Reaktorrohr zwei verschiedene Temperaturzonen von jeweils 800 mm Länge erzeugt. Die Temperatur der ersten Zone (A) betrug 450°C, die der zweiten Zone (B) 220 °C. Die entstandenen Reaktionsgase wurden erst durch eine Wasserwäsche, dann durch eine 10%-NaOH/Wasser-Wäsche, anschließend durch einen mit Molsieb gefüllten Trockenturm geführt und dann auskondensiert. Die entstandenen Produkte wurden gaschromatographisch identifiziert.
Das Reaktionsgemisch (30 g/h) hatte folgende Zusammensetzung (in Gew.-%): 96.0 % 1,1,1,4,4,4-Hexafluorbutan, 1.8 % 1,1,1-Trichlor-2,2,2-trifluorethan, 0.9 % 1-Chlor-2,2,2-trifluorethan, 0.5 % 1,1,1-Trifluorethan, 0.3 % 1,1-Dichlor-2,2,2-trifluorethan, 0.3 % 2-Chlor-1,1,1,4,4,4-hexafluor-buten-2, 0.2 % Sonstige.

Beispiel 2

Die Versuchsdurchführung war analog Beispiel 1, nur daß in der zweiten Reaktorzone statt des Ni/SiO$_2$-Katalysators 0.8 % Rh auf Aktivkohle als Katalysator eingesetzt wurde.
Das Reaktionsgemisch (30 g/h) hatte folgende Zusammensetzung (in Gew.-%): 97.3 % 1,1,1,4,4,4-Hexafluorbutan, 1.3 % 1,1,1-Trichlor-2,2,2-trifluorethan, 0.8 % 1-Chlor-2,2,2-trifluorethan, 0.5 % 1,1,1-Trifluorethan, 0.1 % 1,1-Dichlor-2,2,2-trifluorethan, 0.1 % 2-Chlor-1,1,1,4,4,4-hexafluor-buten-2, 0.1 % Sonstige.

**Patentansprüche**

1. Verfahren zur Herstellung von gesättigten Fluorkohlenwasserstoffen, dadurch gekennzeichnet, daß man mindestens einen Fluorkohlenwasserstoff der Formel (I)

$$R_f\text{-}CH_nCl_cBr_dJ_e \qquad (I)$$

worin    $R_f$ = $C_aF_{(2a-b)+1}H_b$ mit a = 1 - 4, b = 0 - 4,
n = 0 - 2
c = 0 - 3
d = 0 - 3
e = 0 - 3
c + d + e = 3-n ist,

hydrierend dimerisiert, indem man ihn mit Wasserstoff in Gegenwart von mindestens einem Katalysator, der ein Element der I., II. oder VIII. Nebengruppe enthält, in einem Reaktionsgefäß umsetzt, das eine Zone A mit einer Temperatur von 350 bis 500°C und eine Zone B mit einer Temperatur von 150 bis 300°C enthält, wobei man die Reaktanten zuerst durch Zone A und dann durch Zone B leitet.

2. Verfahren nach Anspruch 1, dadurch gekennzeichnet, daß man in beiden Zonen als Katalysator Fe, Co, Cu oder Ni einsetzt.

3. Verfahren nach Anspruch 1, dadurch gekennzeichnet, daß man in beiden Zonen als Katalysator Ni einsetzt.

4. Verfahren nach Anspruch 1, dadurch gekennzeichnet, daß man in Zone A als Katalysator Fe, Co, Cu oder Ni und in Zone B als Katalysator Ni, Ru, Rh, Pd oder Pt einsetzt.

5. Verfahren nach Anspruch 1, dadurch gekennzeichnet, daß man in Zone A als Katalysator Ni und In Zone B als Katalysator Rh einsetzt.

6. Verfahren nach einem der Ansprüche 1 bis 5, dadurch gekennzeichnet, daß in beiden Zonen die Temperatur vom Anfang zum Ende hin abfällt.

7. Verfahren nach einem der Ansprüche 1 bis 6, dadurch gekennzeichnet, daß man als Ausgangsmaterial der Formel (I) 1,1,1-Trichlor-2,2,2-trifluorethan (R113a) einsetzt.

**Claims**

1. A process for the preparation of saturated hydrofluorocarbons, characterized in that at least one fluorocarbon of the formula (I)

$$R_f\text{-}CH_nCl_cBr_dI_e \qquad (I)$$

in which    $R_f$ is $C_aF_{(2a-b)+1}H_b$ where a is 1-4 and
b is 0-4,
n is 0-2
c is 0-3
d is 0-3
e is 0-3
c+d+e is 3-n ,

is reductively dimerized, by reacting it with hydrogen in the presence of at least one catalyst containing an element from subgroups I, II or VIII, in a reaction vessel containing a zone A set at a temperature from 350 to 500 °C and a zone B set at a temperature from 150 to 300 °C, the reactants being passed first through zone A and then through zone B.

2. The process as claimed in claim 1, characterized in that the catalyst used in both zones is Fe, Co, Cu or Ni.

3. The process as claimed in claim 1, characterized in that the catalyst used in both zones is Ni.

4. The process as claimed in claim 1, characterized in that the catalyst used in zone A is Fe, Co, Cu or Ni and the catalyst used in zone B is Ni, Ru, Rh, Pd or Pt.

5. The process as claimed in claim 1, characterized in that the catalyst used in zone A is Ni and the catalyst used in zone B is Rh.

6. The process as claimed in any one of claims 1 to 5, characterized in that the temperature in both zones drops from the beginning toward the end.

7. The process as claimed in any one of claims 1 to 6, characterized in that the formula (I) starting material used is 1,1,1-trichloro-2,2,2-trifluoroethane (R 113a).

**Revendications**

1. Procédé de préparation de fluorocarbures saturés, caractérisé en ce qu'on dimérise par hydrogénation au moins un fluorocarbure répondant à la formule (I)

$$R_f\text{-}CH_nCl_cBr_dI_e \qquad (I)$$

dans laquelle $R_f = C_aF_{(2a+b)+1}H_b$ avec a = 1-4, b = 0-4,
n = 0-2
c = 0-3
d = 0-3
e = 0-3
c+d+e = 3-n ,

en le faisant réagir avec de l'hydrogène, en présence d'au moins un catalyseur comprenant un élément des sous-groupes I, II ou VIII, dans un réacteur comportant une zone A à une température de 350 à 500 °C et une zone B à une température de 150 à 300 °C, les réactifs étant envoyés d'abord dans la zone A et ensuite dans la zone B.

2. Procédé selon la revendication 1, caractérisé en ce que la catalyseur utilisé dans les deux zones est Fe, Co, Cu ou Ni.

3. Procédé selon la revendication 1, caractérisé en ce que la catalyseur utilisé dans les deux zones est Ni.

4. Procédé selon la revendication 1, caractérisé en ce que la catalyseur utilisé dans la zone A est Fe, Co, Cu ou Ni et le catalyseur utilisé dans la zone B est Ni, Ru, Rh, Pd ou Pt.

5. Procédé selon la revendication 1, caractérisé en ce que la catalyseur utilisé dans la zone A est Ni et le catalyseur utilisé dans la zone B est Rh.

6. Procédé selon une quelconque des revendications 1 à 5, caractérisé en ce que, dans les deux zones, la température diminue du début à la fin.

7. Procédé selon une quelconque des revendications 1 à 6, caractérisé en ce que le réactif de départ selon la formule (I) est le 1,1,1-trichloro-2,2,2-trifluoroéthane (R113a).